# EUROPEAN PATENT SPECIFICATION

(11) **EP 0 813 401 B1**
(45) Date of publication and mention of the grant of the patent: **28.06.2000**
(21) Application number: 96907073.9
(22) Date of filing: 01.03.1996
(51) Int. Cl.: A61F 13/15

(54) **COMPOSITE EXHIBITING ELASTIC-LIKE BEHAVIOR**
VERBUNDVLIESSTOFF MIT ELASTIKÄHNLICHEN EIGENSCHAFTEN
COMPOSITE PRESENTANT UNE CERTAINE ELASTICITE

(30) Priority: 03.03.1995 US 397825
(43) Date of publication of application: 29.12.1997
(73) Proprietor: THE PROCTER & GAMBLE COMPANY, Cincinnati, Ohio 45202 (US)
(72) Inventor: WOLF, Scot, Gerald, Cincinnati, OH 45240 (US); BERGMAN, Carl, Louis, Loveland, OH 45140 (US)
(74) Representative: Hirsch, Uwe Thomas
(86) International application number: US9602253
(87) International publication number: WO9627352

(56) References cited:
- WO-A-94/05241
- WO-A-95/03765
- WO-A-95/19258

## Description

### FIELD OF THE INVENTION

The present invention relates to composites, and more particularly, to composites exhibiting an elastic-like behavior in response to an applied and subsequently released (i.e., cycled) elongation along at least one axis thereof.

Composites of the present invention have a wide range of potential uses in both durable and disposable articles, but are particularly well-suited for use in disposable absorbent articles such as sanitary napkins, bandages, pantiliners, disposable diapers, incontinent briefs, diaper holders, training pants, and the like.

### BACKGROUND OF THE INVENTION

Absorbent articles such as sanitary napkins, bandages, pantiliners, disposable diapers, incontinent briefs, diaper holders and training pants are designed to absorb and retain liquid and other discharges from the human body and to prevent body and clothing soiling. Typically, most absorbent articles are made of materials that will not readily stretch under the forces that the absorbent article is normally subjected to when worn. The inability of the materials comprising the absorbent article to stretch when subjected to normal wearing forces causes the absorbent article to have certain drawbacks. One drawback is the lack of comfort for the wearer. The wearer should ideally be able to notice a difference between an absorbent article that stretches to conform to the wearer's body with the wearer's movements and an absorbent article that fails to stretch.

Several attempts have been made to make one or more components or portions of absorbent articles stretchable in response to relatively low wearing forces exerted upon the absorbent articles. Typically, prior art solutions rely on the addition of traditional elastics such as natural or synthetic rubber. For example, traditional elastics have been secured to portions of the topsheet and/or backsheet of absorbent articles, such as in the waist portion of a disposable diaper, to provide a better fit and overall comfort for the wearer. However, traditional elastics are costly and require a certain degree of manipulation and handling during assembly. While traditional elastics do provide a degree of stretch for the absorbent article, the materials to which the traditional elastics is secured are typically not normally considered elastic or stretchable. Therefore, the added traditional elastics must be prestretched prior to being secured to the material or the material must be subjected to mechanical processing, e.g., ring-rolling, to permanently elongate the material to extend beyond its initial untensioned length and allow the added traditional elastic to be effective. Otherwise, the added traditional elastic is restrained by the material and is rendered inoperable.

Accordingly, it is an object of the present invention to provide a composite which exhibits an "elastic-like" behavior in a direction of applied elongation without the use of costly traditional elastic materials. As used herein, the term "elastic-like" describes the behavior of a composite which when subjected to an applied elongation, the composite extends in the direction of applied elongation and when the applied elongation is released the composite returns, to a substantial degree, to its untensioned condition. While composite web materials exhibiting an elastic-like behavior have a wide range of utility, e.g., durable articles of apparel, disposable articles of apparel, covering materials such as upholstery, wrapping materials for complex shapes and the like, they are particularly well suited for use in disposable absorbent articles.

### SUMMARY OF THE INVENTION

The present invention pertains to a composite exhibiting an elastic-like behavior in response to an applied axial elongation along at least one axis thereof. The composite comprises a first web material in a relaxed condition bonded to a stretched second web material. Preferably, the first web material is intermittently bonded to the second web material in a "column" type pattern. The second web material comprises a strainable network having first and second regions formed of substantially the same material composition. The first region provides a first, elastic-like resistive force to the applied axial elongation, and the second region provides a second distinctive resistive force to further applied axial elongation, thereby providing at least two stages of resistive forces in use.

The composite is particularly well-suited for use in disposable absorbent articles such as sanitary napkins, bandages, pantiliners, disposable diapers, incontinent briefs, diaper holders, training pants and the like. The composite is particularly useful in the contractible and extensible portions of a disposable diaper, e.g., the extensible side panels, elastic waist features, elasticized leg cuffs, fastening member, absorbent core, and backsheet.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which like designations are used to designate substantially identical elements and in which:
FIG. 1 is a plan view illustration of a disposable diaper embodiment of the present invention having portions cut away to reveal underlying structure, and with the inner surface of the diaper facing the viewer;
FIG 2 is a plan view illustration of a preferred embodiment of a composite web of the present invention having a portion of the second web cut away to reveal the underlying first web;
FIGS. 2A -2C are segmented, perspective illustrations of the second web of the composite illustrated in FIG. 2, subjected to varying degrees of elongation; and
FIG. 3 is a fragmentary plan view of a diaper of the present invention in the first waist region showing the elements of the elastic waist feature and the extensible side panels.

### DETAILED DESCRIPTION OF THE INVENTION

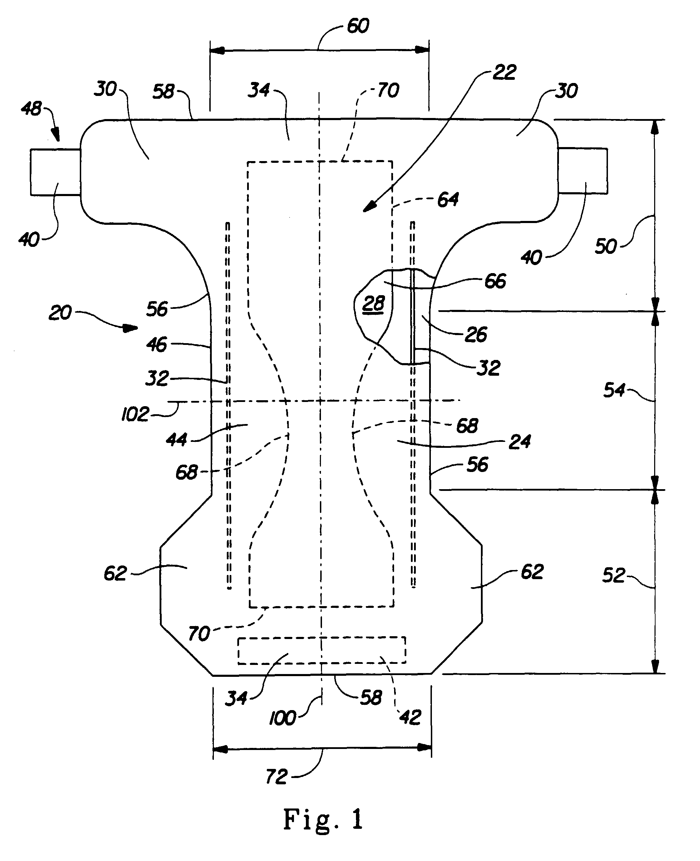

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A preferred embodiment of an absorbent article is the disposable absorbent article, diaper 20, shown in Figure 1. As used herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments, sanitary napkins, training pants, and the like.

Figure 1 is a plan view of the diaper 20 in its flat-out, uncontracted state (i.e., with elastic induced contraction pulled out) with portions of the structure being cut-away to more clearly show the construction of the diaper 20 and with the portion of the diaper 20 which faces or contacts the wearer, the inner surface, oriented towards the viewer. As shown in Figure 1, the diaper 20 preferably comprises a containment assembly 22 comprising a liquid pervious topsheet 24; a liquid impervious backsheet 26 joined with the topsheet 24; and an absorbent core 28 positioned between the topsheet 24 and the backsheet 26. The diaper further comprises elasticized leg cuffs 32; ear flaps 62; elastic waist features 34; a fastening system 48 comprising a pair of first fastening members 40 and a second fastening member 42; and extensible side panels 30.

The diaper 20 also has two centerlines, a longitudinal centerline 100 and a transverse centerline 102. The term "longitudinal", as used herein, refers to a line, axis, or direction in the plane of the diaper 20 that is generally aligned with (e.g. approximately parallel with) a vertical plane which bisects a standing wearer into left and right halves when the diaper 20 is worn. The terms "transverse" and "lateral", as used herein, are interchangeable and refer to a line, axis or direction which lies within the plane of the diaper that is generally perpendicular to the longitudinal direction (which divides the wearer into front and back body halves).

The diaper 20 is shown in Figure 1 to have an inner surface 44 (facing the viewer in Figure 1), an outer surface 46 opposed to the inner surface 44, a first waist region 50, a second waist region 52 opposed to the first waist region 50, a crotch region 54 positioned between the first waist region 50 and the second waist region 52, and a periphery which is defined by the outer perimeter or edges of the diaper 20 in which the longitudinal edges are designated 56 and the end edges are designated 58. The inner surface 44 of the diaper 20 comprises that portion of the diaper 20 which is positioned adjacent to the wearer's body during use (i.e., the inner surface 44 generally is formed by at least a portion of the topsheet 24 and other components joined to the topsheet 24). The outer surface 46 comprises that portion of the diaper 20 which is positioned away from the wearer's body (i.e., the outer surface 46 is generally formed by at least a portion of the backsheet 26 and other components joined to the backsheet 26). As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. The first waist region 50 and the second waist region 52 extend from the end edges 58 of the periphery to the crotch region 54. The first waist region 50 comprises a central region 60 and a pair of extensible side panels 30 which typically comprise the outer lateral portions of the first waist region 50. The second waist region 52 comprises a central region 72 and a pair of ear flaps 62 which typically comprise the outer lateral portions of the second waist region 52. The extensible side panels positioned in the first waist region 50 are designated 30 while the ear flaps in the second waist region 52 are designated 62.

The containment assembly 22 of the diaper 20 is shown in Figure 1 as comprising the main body (chassis) of the diaper 20. The containment assembly 22 comprises at least an absorbent core 28 and preferably an outer covering layer comprising the topsheet 24 and the backsheet 26. When the absorbent article comprises a separate holder and a liner, the containment assembly 22 generally comprises the holder and the liner (i.e., the containment assembly 22 comprises one or more layers of material to define the holder while the liner comprises an absorbent composite such as a topsheet, a backsheet, and an absorbent core.) Generally, the containment assembly 22 comprises the main structure of the diaper with other features added to form the composite diaper structure. Thus, the containment assembly 22 for the diaper 20 generally comprises the topsheet 24, the backsheet 26, and the absorbent core 28.

Figure 1 shows a preferred embodiment of the containment assembly 22 in which the topsheet 24 and the backsheet 26 have length and width dimensions generally larger than those of the absorbent core 28. The topsheet 24 and the backsheet 26 extend beyond the edges of the absorbent core 28 to thereby form the periphery of the diaper 20. While the topsheet 24, the backsheet 26, and the absorbent core 28 may be assembled in a variety of well known configurations, preferred containment assembly configurations are described generally in U.S. Patent 3,860,003 entitled "Contractible Side Portions for Disposable Diaper" which issued to Kenneth B. Buell on January 14, 1975; and U.S. Patent 5,151,092 entitled "Absorbent Article With Dynamic Elastic Waist Feature Having A Predisposed Resilient Flexural Hinge" which issued to Kenneth B. Buell et al., on September 29, 1992.

The absorbent core 28 may be any absorbent member which is generally compressible, conformable, and non-irritating to the wearer's skin, and capable of absorbing and retaining liquids such as urine and other certain body exudates. As shown in Figure 1, the absorbent core 28 has an outer surface 64, an inner surface 66, side edges 68, and waist edges 70. The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 28 may vary (e.g., the absorbent core may have varying caliper zones, a hydrophilic gradient, a superabsorbent gradient, or lower average density and lower average basis weight acquisition zones; or may comprise one or more layers or structures). However, the total absorbent capacity of the absorbent core 28 should be compatible with the design loading and the intended use of the diaper 20. The size and absorbent capacity of the absorbent core 28 may also be varied to accommodate wearers ranging from infants through adults.

One embodiment of the diaper 20 has an asymmetric, modified T-shaped, absorbent core 28 having ears in the first waist region and a generally rectangular shape in the second waist region. Exemplary absorbent structures for use as the absorbent core 28 of the present invention that have achieved wide acceptance and commercial success are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989. The absorbent core may further comprise the dual core system containing acquisition/distribution core of chemically stiffened fibers positioned over the absorbent storage cores as detailed in U.S. Patent 5,234,423, entitled "Absorbent Article With Elastic Waist Feature and Enhanced Absorbency" issued to Alemany et al., on August 10, 1993; and in U.S. Patent 5,147,345, entitled "High Efficiency Absorbent Articles For Incontinence Management" issued to Young, LaVon and Taylor on September 15, 1992.

The backsheet 26 is positioned adjacent the outer surface 64 of the absorbent core 28 and is preferably joined thereto by attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. An example of a suitable attachment means comprising an open pattern network of filaments of adhesive is disclosed in U.S. Patent 4,573,986 entitled "Disposable Waste-Containment Garment", which issued to Minetola et al. on March 4, 1986. Another suitable attachment means comprising several lines of adhesive filaments swirled into a spiral pattern is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the diaper 20 such as bedsheets and undergarments. Further, the backsheet 26 may permit vapors to escape from the absorbent core 28 (i.e., breathable) while still preventing exudates from passing through the backsheet 26. Thus, the backsheet 26 may comprise a woven or nonwoven material, polymeric films such as thermoplastic films of polyethylene or polypropylene, or composite materials such as a film-coated nonwoven material. An example of a suitable backsheet is a thermoplastic film having a thickness of from about 0.012 mm (0.5 mil) to about 0.051 mm (2.0 mils). Other suitable materials for the backsheet 26 include RR8220 blown films and RR5475 cast films as manufactured by Tredegar Industries, Inc. of Terre Haute, IN. The backsheet 26 is preferably embossed and/or matte finished to provide a more clothlike appearance. Another suitable backsheet is a laminate comprising a thermoplastic film secured to a nonwoven web of staple fibers.

The topsheet 24 is positioned adjacent the inner surface 66 of the absorbent core 28 and is preferably joined thereto and to the backsheet 26 by attachment means (not shown) such as those well known in the art. Suitable attachment means are described with respect to joining the backsheet 26 to the absorbent core 28. In a preferred embodiment of the present invention, the topsheet 24 and the backsheet 26 are joined directly to each other in the diaper periphery and are indirectly joined together by directly joining them to the absorbent core 28 by the attachment means (not shown).

The topsheet 24 is compliant, soft feeling, and non-irritating to the wearer's skin. Further, the topsheet 24 is preferably liquid pervious permitting liquids (e.g., urine) to readily penetrate through its thickness. A suitable topsheet 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. The topsheet 24 is preferably made of a hydrophobic material to isolate the wearer's skin from liquids which have passed through the topsheet 24 and are contained in the absorbent core 28 (i.e. to prevent rewet). If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 28. The topsheet 24 can be rendered hydrophilic by treating it with a surfactant. Suitable methods for treating the topsheet 24 with a surfactant include spraying the topsheet 24 material with the surfactant and immersing the material into the surfactant. A more detailed discussion of such a treatment and hydrophilicity is contained in U.S. Patents 4,988,344 entitled "Absorbent Articles with Multiple Layer Absorbent Layers" issued to Reising, et al on January 29, 1991 and U.S. Patent 4,988,345 entitled "Absorbent Articles with Rapid Acquiring Absorbent Cores" issued to Reising on January 29, 1991.

There are a number of manufacturing techniques which may be used to manufacture the topsheet 24. For example, the topsheet 24 may be a nonwoven web of fibers. When the topsheet 24 comprises a nonwoven web, the web may be spunbonded, carded, wet-laid, melt-blown, hydroentangled, combinations of the above, or the like. A suitable topsheet 24 is carded and thermally bonded by means well known to those skilled in the fabrics art. A satisfactory topsheet 24 comprises staple length polypropylene fibers having a denier of about 2.2 As used herein, the term "staple length fibers" refers to those fibers having a length of at least about 15.9 mm (0.625 inches). Preferably, the topsheet 24 has a basis weight from about 18 to about 25 grams per square meter. A suitable topsheet is manufactured by Veratec, Inc., a Division of International Paper Company, of Walpole, Mass. under the designation P-8.

The diaper 20 preferably further comprises elasticized leg cuffs 32 for providing improved containment of liquids and other body exudates. Each elasticized leg cuff 32 may comprise several different embodiments for reducing the leakage of body exudates in the leg regions. (The leg cuff can be and is sometimes also referred to as leg bands, side flaps, barrier cuffs, or elastic cuffs.) U.S. Patent 3,860,003 describes a disposable diaper which provides a contractible leg opening having a side flap and one or more elastic members to provide an elasticized leg cuff (gasketing cuff). U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987, describes a disposable diaper having dual cuffs including a gasketing cuff and a barrier cuff. While each elasticized leg cuff 32 may be configured so as to be similar to any of the leg bands, side flaps, barrier cuffs, or elastic cuffs described above, each elasticized leg cuff 32 preferably comprises a gasketing cuff as described in the above-referenced U.S. Patent 3,860,003.

The diaper 20 preferably further comprises an elastic waist feature 34 that helps provide improved fit and containment. The elastic waist feature 34 is that portion or zone of the diaper 20 which is intended to elastically expand and contract to dynamically fit the wearer's waist. The elastic waist feature 34 preferably extends longitudinally outwardly from at least one of the waist edges 70 of the absorbent core 28 and generally forms at least a portion of the end edge 58 of the diaper 20. Alternatively, the elastic waist feature 34 may overlap one of the waist edges 70 of the absorbent core 28. Disposable diapers are generally constructed so as to have two elastic waist features, one positioned in the first waist region 50 and one positioned in the second waist region 52, although diapers can be constructed with a single elastic waist feature. If a diaper has two elastic waist features, they may be identical to one another or different from each other depending on the desired fit and containment properties. Further, while the elastic waist feature 34 or any of its constituent elements can comprise a separate element affixed to the diaper 20, the elastic waist feature 34 may be constructed as an extension of other elements of the diaper such as the backsheet 26 or the topsheet 24, preferably both the backsheet 26 and the topsheet 24. The waist feature 34 may be constructed in a number of different configurations including those described in U.S. Patent 4,515,595 issued to Kievit et al. on May 7, 1985; U.S. Patent 5,026,364 issued to Robertson on June 25, 1991; and the above referenced U.S. Patent 5,151,092 issued to Buell;

In a preferred embodiment of the present invention, the diaper 20 also comprises ear flaps 62 that extend laterally outwardly from each longitudinal edge 56 of the containment assembly 22 in the second waist region 52. The ear flaps 62 provide a structure to which the first waist region 50 can be attached to encircle the legs and waist of the wearer. The ear flaps 62 may take on a number of different sizes, shapes, configurations, and materials. The ear flaps 62 may comprise a portion of the material making up one or more of the diaper elements, including the topsheet 24, and the backsheet 26. Alternatively, the ear flaps 62 may comprise a separate element or a plurality of elements joined to the diaper. Suitable materials for use as the ear flaps 62 include woven webs; nonwoven webs; films, including polymeric films; foams; laminate materials including film laminates, nonwoven laminates, or zero strain laminates; elastomers; composites; or any combination of materials herein described or as described with respect to the extensible side panels as are known in the art. The ear flaps 62 may be joined to the containment assembly 22 by any means as known in the art; for example the ear flaps 62 may be continuously or intermittently bonded to the containment assembly using heated or unheated adhesive, heat bonding, pressure bonding, ultrasonic bonding, dynamic mechanical bonding or any other method that is known in the art.

The diaper 20 additionally comprises an extensible side panel 30 disposed adjacent each longitudinal edge 56 of the containment assembly 22 of the diaper 20, preferably in the first waist region 50. (As used herein, the term "disposed" means that an element(s) of the absorbent article is formed (joined and positioned) in a particular place or position as a unitary structure with other elements of the absorbent article or as a separate element joined to another element of the absorbent article.) The extensible side panels 30 provide an elastically extensible feature that provides a more comfortable and contouring fit by initially conformably fitting the diaper to the wearer and sustaining the fit throughout the time of wear well past when the diaper has been loaded with exudates since the extensible side panels 30 allow the sides of the diaper 20 to expand and contract. The extensible side panels 30 further provide more effective application of the diaper since even if the diaperer fits the diaper to the wearer asymmetrically, the diaper will "self-adjust" during wear to attain an improved fit. The extensible side panels 30 of the present invention also provide improved dynamic fit about the waist as well as the thigh of the wearer, reducing the possibility of sagging and gapping both at the waist and about the leg that can cause leakage, while increasing freedom of motion and wearer comfort in each area.

While the diaper 20 of the present invention preferably has extensible side panels 30 disposed in the first waist region 50; alternatively, the diaper 20 may be provided with extensible side panels 30 disposed in the second waist region 52 or in both the first waist region 50 and the second waist region 52. While the extensible side panels 30 may be constructed in a number of configurations, examples of diapers with extensible side panels are disclosed in U.S. Patent 4,857,067, entitled "Disposable Diaper Having Shirred Ears" issued to Wood, et al. on August 15, 1989; U.S. Patent 4,381,781 issued to Sciaraffa, et al. on May 3, 1983; U.S. Patent 4,938,753 issued to Van Gompel, et al. on July 3, 1990; and U.S. Patent Application Serial No. 08/155,048 entitled "Absorbent Article with Multi-Directional Extensible Side Panels", filed in the name of Miguel A. Robles, et al. on November 19, 1993.

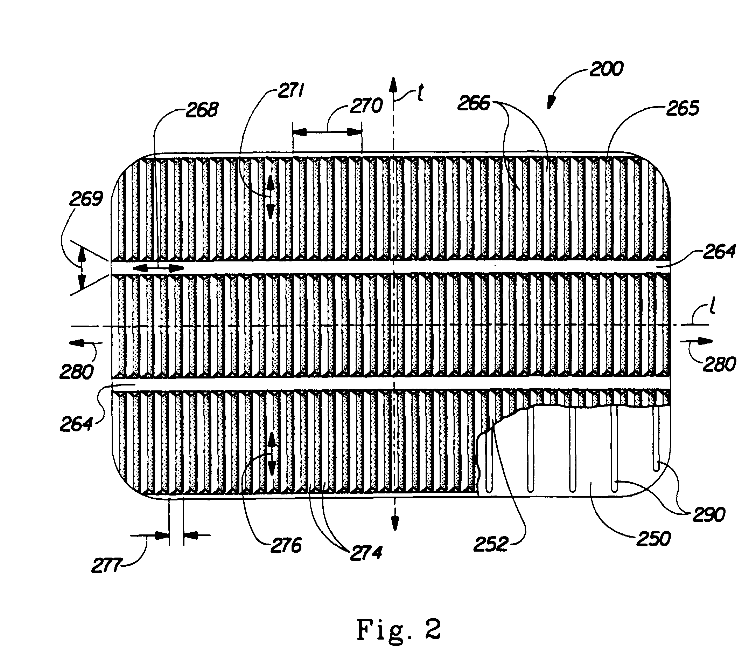

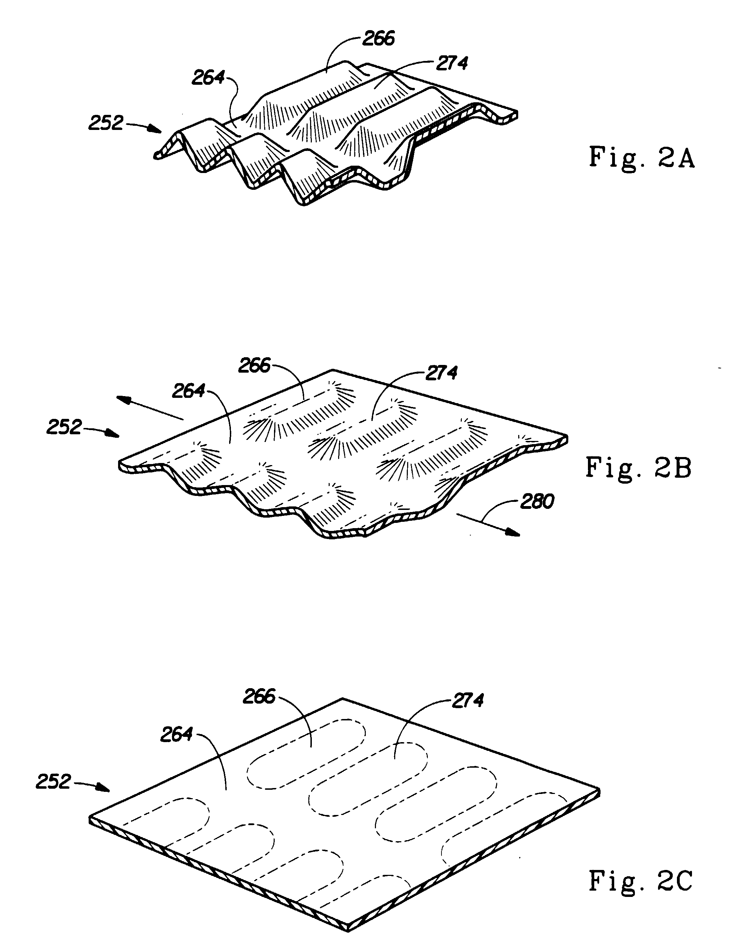

As mentioned above, portions of the diaper 20, (e.g., extensible side panels 30, elastic waist features 34, and elasticized leg cuffs 32), may be constructed from extensible and contractible materials known in the art. In certain embodiments it may also be desirable to have other portions of the diaper, such as the absorbent core, backsheet, topsheet, fastening system and/or the entire diaper be constructed from extensible and contractible materials known in the art. Preferably, the composite of the present invention, generally indicated as 200 in Figure 2, is used to render portions or the entire diaper extensible and contractible. The composite 200 is preferably comprised of at least two web materials, first web material 250 and second web material 252, bonded to one another at 290 in a regular or irregular pattern. First web material 250 and second web material 252 may be the same size or different sizes, (e.g., they may have similar or different length, width and thickness dimensions). The composite 200 may also comprise three or more webs if so desired. For example, additional webs may be added to improve comfort, increase bulk, and increase resiliency of the composite. The term "web", as used herein, refers to a sheet-like material comprising a single layer of material or a laminate of two or more layers.

Figure 2 is a plan view illustration of one embodiment of a composite according to the present invention. The embodiment shown in Figure 2 comprises a first web material 250 in a relaxed condition and a second structural elastic-like film (SELF) web material 252 in a stretched or elongated condition. Examples of SELF webs are disclosed in U.S. PATENT 5 518 801. In the embodiment illustrated in Figure 2, the first and second web materials 250 and 252 are each constructed of a single layer of material. Alternatively, the first and/or second web materials 250 and 252 may each be constructed of two or more layers.

The SELF web 252 has two centerlines, a longitudinal centerline, 1, and a transverse or lateral centerline, t, which is generally perpendicular to the longitudinal centerline. The SELF web 252 is preferably comprised of substantially of linear low density polyethylene (LLDPE) although it may also be comprised of other polyolefins such as polyethylenes including low density polyethylenes (LDPE), ultra low density polyethylene (ULDPE), high density polyethylene (HDPE), or polypropylene and/or blends thereof of the above and other materials. Examples of other suitable polymeric materials include, but are not limited to, polyesters, polyurethanes, compostable or biodegradable polymers, and breathable polymers.

The SELF web 252 includes a "strainable network" of distinct regions. As used herein, the term "strainable network" refers to an interconnected and interrelated group of regions which are able to be extended to some useful degree in a predetermined direction providing the SELF web with an elastic-like behavior in response to an applied and subsequent released elongation. The strainable network includes at least a first region 264 and a second region 266. The SELF web also includes transitional regions 265 which are at the interface between the first regions 264 and the second regions 266. The transitional regions 265 will generally exhibit complex combinations of behavior of both the first region and the second region. It is recognized that every embodiment of SELF webs will have transitional regions, however, the SELF webs are largely defined by the behavior of the SELF web material in the distinctive regions (e.g., the first region and the second region). Therefore, the ensuing description will be concerned with the behavior of the SELF web material in the first regions and the second regions since the SELF web is not significantly dependent upon the complex behavior of the transitional regions.

The SELF web 252 has a first surface and an opposing second surface. In the embodiment shown in Figure 2, the strainable network includes a plurality of first regions 264 and a plurality of second regions 266. The first regions 264 have a first axis 268 and a second axis 269, wherein the first axis 268 is preferably longer than the second axis 269. The first axis 268 of the first region 264 is substantially parallel to the longitudinal axis of the SELF web while the second axis 269 is substantially parallel to the transverse axis of the SELF web. Preferably, the dimension of the second axis 269 of the first region, (i.e., the width of the first region), is from about 0.0254 cm (0.01 inches) to about 0.5 inches, and more preferably from about 0.0762 cm (0.03 inches) to about 0.635 cm (0.25 inches). The second regions 266 have a first axis 270 and a second axis 271. The first axis 270 is substantially parallel to the longitudinal axis of the SELF web, while the second axis 271 is substantially parallel to the transverse axis of the SELF web. Preferably, the dimension of the second axis 271 of the second region, (i.e., the width of the second region), is from about 0,0254 cm (0.01 inches) to about 5,08 cm (2.0 inches) and more preferably, from about 0.3175 cm - (0.125 inches) to about 2,54 cm (1.0 inches). In the preferred embodiment of Figure 2, the first regions 264 and the second regions 266 are substantially linear, extending continuously in a direction substantially parallel to the longitudinal axis of the SELF web.

The first region 264 has an elastic modulus E1 and a cross-sectional area A1. The second region 266 has an elastic modulus E2 and a cross-sectional area A2.

In the illustrated embodiment, a portion of each SELF web has been "formed" such that the SELF web exhibits a resistive force along an axis, which in the case of the illustrated embodiment is substantially parallel to the longitudinal axis "1" of the SELF web, when subjected to an applied axial elongation in a direction substantially parallel to the longitudinal axis. As used herein, the term "formed" refers to the creation of a desired structure or geometry upon the SELF web that will substantially retain the desired structure or geometry when it is not subjected to any externally applied elongations or forces. A SELF web of the present invention is preferably comprised of at least a first region and a second region, wherein the first region is visually distinct from the second region. As used herein, the term "visually distinct" refers to features of the SELF web material which are readily discernible to the normal naked eye when the SELF web material or objects embodying these SELF web material are subjected to normal use. Preferably, the first region has a "surface-pathlength" less than that of the second region, as measured parallel to a predetermined axis when the material is in an untensioned state. As used herein, the term "surface-pathlength" refers to a measurement along the topographic surface of the region in question in a direction parallel to an axis. The method for determining the surface-pathlength of the respective regions is set forth in U.S. PATENT 5 518 801.

Methods for forming SELF web materials include, but are not limited to, embossing by mating plates or rolls, thermoforming, high pressure hydraulic forming, or casting. While the entire portion of the SELF web has been subjected to a forming operation, the present invention may also be practiced by subjecting to formation only a portion of the web.

In the preferred embodiment shown in Figures 2 and 2A, the first regions 264 are substantially planar. That is, the material within the first region 264 is in substantially the same condition before and after the formation step undergone by the SELF web. The second regions 266 include a plurality of raised rib-like elements 274. The rib-like elements 274 may be embossed, debossed or a combination thereof. The rib-like elements 274 have a first or major axis 276 which is substantially parallel to the transverse axis of the SELF web and a second or minor axis 277 which is substantially parallel to the longitudinal axis of the SELF web. The first axis 276 of the rib-like elements 274 is at least equal to, and preferably longer than the second axis 277. Preferably, the ratio of lengths of the first axis 276 to the second axis 277 is at least about 1:1, or greater, and more preferably at least about 2:1 or greater.

The rib-like elements 274 in the second region 266 may be separated from one another by unformed areas, essentially unembossed or debossed, or simply formed as spacing areas. Preferably, the rib-like elements 274 are adjacent one another and are separated by an unformed area of less than 0,254 cm (0.10 inches) as measured perpendicular to the major axis 276 of the rib-like element 274, and more preferably, the rib-like elements 274 are contiguous having no unformed areas between them.

The first region 264 and the second region 266 each have a "projected pathlength". As used herein, the term "projected pathlength" refers to length of a shadow of a region that would be thrown by parallel light. The projected pathlength of the first region 264 and the projected pathlength of the second region 266 are equal to one another.

The first region 264 has a surface-pathlength, L1, which is less than the surface-pathlength, L2, of the second region 266 as measured topographically in a direction parallel to the longitudinal axis of the SELF web while the SELF web is in an untensioned condition. Preferably, the surface-pathlength of the second region 266 is at least about 15% greater than that of the first region 264, more preferably at least about 30% greater than that of the first region, and most preferably at least about 70% greater than that of the first region. In general, the greater the surface-pathlength of the second region, the greater will be the elongation of the SELF web before encountering the force wall.

What makes the SELF web particularly well suited for use in absorbent articles is that it exhibits a modified "Poisson lateral contraction effect" substantially less than that of an otherwise identical unformed base web of similar material composition. As used herein, the term "Poisson lateral contraction effect" describes the lateral contraction behavior of a material which is being subjected to an applied elongation. Preferably, the Poisson lateral contraction effect of the SELF web of the present invention is less than about 0.4 when the SELF web is subjected to about 20% elongation. The method for determining the Poisson lateral contraction effect is set forth in U.S. PATENT 5 518 801. Preferably, the SELF web exhibits a Poisson lateral contraction effect less than about 0.4 when the composite SELF web is subjected to about 40, 50 or even 60% elongation. The Poisson lateral contraction effect of the SELF webs is determined by the amount of the web material which is occupied by the first and second regions, respectively. As the area of the SELF web occupied by the first region increases, the Poisson lateral contraction effect also increases. Conversely, as the area of the SELF web occupied by the second region increases the Poisson lateral contraction effect decreases. Preferably, the percent area of the SELF web occupied by the first region is from about 2% to about 90%, and more preferably from about 5% to about 50%.

Web materials of the prior art which have at least one layer of an elastomeric material will generally have a large Poisson lateral contraction effect, i.e., they will "neck down" as they elongate in response to an applied force. SELF web materials of the present invention can be designed to moderate if not substantially eliminate the Poisson lateral contraction effect.

For the SELF web 252 and the composite web 200, the direction of applied axial elongation, D, indicated by arrows 280 in FIG. 2, is substantially perpendicular to the first axis 276 of the rib-like elements 274. The rib-like elements 274 are able to unbend or geometrically deform in a direction substantially perpendicular to their first axis 276 to allow extension in the SELF web 252 and therefore extension of the composite web 200.

Referring now to Figure 2B, as the SELF web 252 is subjected to an applied axial elongation, D, indicated by arrows 280 in Figures 2 and 2B, the first regions 264 having the shorter surface-pathlength, L1, provide most of the initial resistive force, P1, as a result of molecular-level deformation, to the applied elongation. Meanwhile, the rib-like elements 274 in the respective second regions 266 are experiencing geometric deformation, or unbending, and offer minimal resistance to the applied elongation. As the axial elongation increases, the rib-like elements 274 approach alignment with the applied elongation. That is, the second region is exhibiting a change from geometric deformation to molecular-level deformation. This is the onset of the "force wall" as used herein, the term "force wall", refers to the behavior of the resistive force of a material during elongation wherein at some point, the force resisting the applied elongation suddenly increases. Referring now to Figure 2C, the rib-like elements 274 have become substantially aligned with the axis of applied elongation (i.e., the second region has reached its limit of geometric deformation) and begin to resist further elongation via molecular-level deformation. The second region 266 now contributes, as a result of molecular-level deformation, a second resistive force, P2, to further applied elongation. The resistive forces to elongation, i.e., the molecular-level deformation of the first region 264 and the molecular-level deformation of the second regions 266, provide a total resistive force, PT, which is greater than the resistive force provided by the molecular-level deformation of the first region 264 and the geometric deformation of the second region 266. Force elongation curves depicting the behavior of the SELF web and the method for generating the force-elongation curves is set forth in U.S. PATENT 5 518 801.

The maximum elongation occurring while the second region experiences geometric deformation is referred to as the "available stretch" of the SELF web. The available stretch is often found to be of interest in disposable absorbent articles, and can be largely controlled by the extent to which the surface-pathlength of the second region exceeds the surface-pathlength of the first region and the composition of the component webs.

When the SELF web is subjected to an applied elongation, the SELF web exhibits an elastic-like behavior as it extends in the direction of applied elongation and returns to its substantially untensioned condition once the applied elongation is removed, unless the SELF web is extended beyond the point of yielding. The SELF web is able to undergo multiple cycles of applied elongation without losing its ability to substantially recover. Accordingly, the SELF web is able to return to its substantially untensioned condition once the applied elongation or force is removed. The elastic hysteresis curves for the SELF web and the method for generating the elastic hysteresis curves is set forth in U.S. PATENT 5 518 801.

Similar to the SELF web, the composite web, which includes the first web in a relaxed condition bonded to the stretched second SELF web, exhibits an elastic-like behavior as it extends in the direction of applied elongation and returns to its substantially untensioned condition once the applied elongation is removed. The composite is able to undergo multiple cycles of applied elongation without losing its ability to substantially recover. Accordingly, the composite is able to return to its substantially untensioned condition once the applied elongation is removed.

While the SELF web may be easily and reversibly extended in the direction of applied axial elongation, in a direction substantially perpendicular to the first axis of the rib-like elements, the SELF web is not as easily extended in a direction substantially parallel to the first axis of the rib-like elements. The formation of the rib-like elements allows the rib-like elements to geometrically deform in a direction substantially perpendicular to the first or major axis of the rib-like elements, while requiring substantially molecular-level deformation to extend in a direction substantially parallel to the first axis of the rib-like elements.

The amount of applied force required to extend the SELF web is dependent upon the composition and cross-sectional area of the web materials forming the SELF web and the width and spacing of the first regions, with narrower and more widely spaced first regions requiring lower applied extension forces to achieve the desired elongation. The first axis, (i.e., the length) of the first regions is preferably greater than the second axis, (i.e., the width) of the first region with a preferred length to width ratio of from about 5:1 or greater.

The depth and frequency of rib-like elements can also be varied to control the available stretch of the SELF web. The available stretch is increased if for a given frequency of rib-like elements, the height or degree of deformation imparted on the rib-like elements is increased. Similarly, the available stretch is increased if for a given height or degree of deformation, the frequency of rib-like elements is increased.

The first and second web materials may be comprised of polyolefins such as polyethylenes, including linear low density polyethylene (LLDPE), low density polyethylene (LDPE), ultra low density polyethylene (ULDPE), high density polyethylene (HDPE), or polypropylene and blends thereof with the above and other materials. Examples of other suitable polymeric materials which may also be used include, but are not limited to, polyester, polyurethanes, compostable or biodegradable polymers, heat shrink polymers, thermoplastic elastomers, metallocene catalyst-based polymers (e.g., INSITE® available from Dow Chemical Company and EXXACT® available from Exxon), and breathable polymers. The web materials may also be comprised of a synthetic woven, synthetic knit, nonwoven, apertured film, macroscopically expanded three-dimensional formed film, absorbent or fibrous absorbent material, foam filled composition or laminates and/or combinations thereof. The nonwovens may be made but not limited to any of the following methods: spunlace, spunbond, meltblown, carded and/or air-through or calender bonded, with a spunlace material with loosely bonded fibers being the preferred embodiment.

The first and second web materials may be made from two-dimensional apertured films and macroscopically expanded, three-dimensional, apertured formed films. Examples of macroscopically expanded, three-dimensional, apertured formed films are described in U.S. Patent 3,929,135 issued to Thompson on December 30, 1975; U.S. Patent 4,324,246 issued to Mullane, et al. on April 13, 1982; U.S. Patent 4,342,314 issued to Radel, et al. on August 3, 1982; U.S. Patent 4,463,045 issued to Ahr, et al. on July 31, 1984; and U.S. Patent 5,006,394 issued to Baird on April 9, 1991.

The first and second web materials may comprise laminates of apertured films and nonwoven materials whereby in the process of forming such materials, the connections between a plurality of the nonwoven fibers are broken up to protrude slightly through the apertures of the apertured film.

It may be desirable in certain embodiments to have the composite web exhibit a certain degree of bulkiness and bending resistance. Laminates of polymer films with high-loft nonwoven materials, and laminates with multi-layers of nonwovens are ways of providing increased bulk. Other methods for creating bulk include the formation of a single layer of polymer film in the manner of this invention followed by prestretching of the film and subsequent application of the nonwoven to one or both sides while the polymer film is in its prestretched condition. Upon relaxation of the stretch, the nonwoven material forms puckers which give the material added bulk. Another method for making bulky laminates is by forming individual polymeric film layers in the manner of this invention, followed by lamination of multiple layers of these materials. Three dimensionally apertured films that have been formed using the method described herein also provide good bulk in a laminate structure.

Other materials which may be subject to the deformation processes disclosed herein for producing webs which exhibit an elastic-like behavior in the direction of applied force include polymeric foams and thermally bonded air-laid fibrous structures.

The first web material may be a pre-strained web material. That is, prior to being bonded to the second web material, the first web material may be strained and subsequently relaxed.

Referring again to Figure 2, the first web 250 while in a relaxed condition is intermittently bonded at 290 to the second SELF web 252, which is in a stretched or elongated condition to form the composite 200. Preferably, the first web 250 is stretched from about 5% to about 500% of its original length, more preferably from about 10% to about 250% of its original length, and most preferably from about 25% to about 100% of its original length. The first and second webs 250 and 252 may be intermittently bonded together in a regular or irregular pattern. In the embodiment shown in FIG. 2, the first web 250 is intermittently bonded to the second web 252 with a regular pattern of adhesive beads, lines, or columns 290 oriented generally parallel to axis 271. Alternatively, the first and second webs may be intermittently bonded together with spirals or spots of adhesive. Bonding may be effected by any conventional means such as heat sealing, sonic bonding, etc. Intermittent bonding of the component webs creates unglued or open areas in the combined composite allowing the composite to expand and stretch in an elastic-like manner. Conversely, in a substantially continuously bonded composite, the webs are bonded substantially continuously to one another throughout their areas of interface.

After the first and second webs 250 and 252 have been intermittently bonded together to form the composite 200, the second SELF web 252 may have sufficient strength to pucker or gather the composite when the tension is released. The puckering provides bulk and softness in the composite which may be desirable when used as a portion of a disposable absorbent article. When intermittently bonded together with parallel beads or columns of adhesive, the composite will have a plurality of rugosities which provide both bulk and bending resistance.

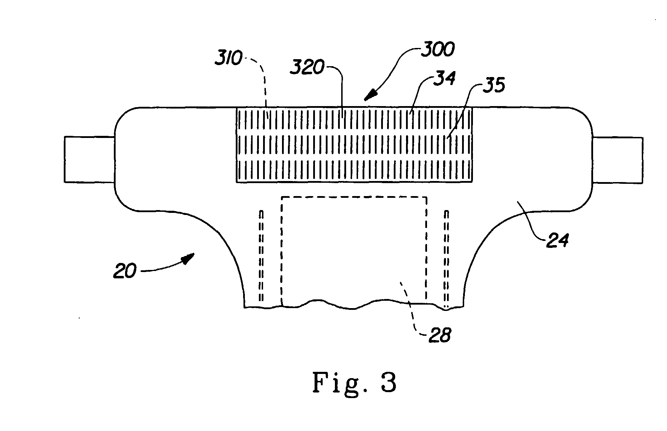

Referring now to Figure 3, the elastic waist feature 34 comprising an elastic waistband 35 in the first waist region 50 comprises a composite indicated generally as 300. Composite 300 includes a first component 310 and a second component 320. The first component 310, in a relaxed condition, is intermittently bonded to the second component 320, while the second component 320 is in a stretched or elongated condition. The first component 310 comprises a portion of the topsheet 24 and a portion of the backsheet 26. The second component 320 is preferably a SELF web comprised of a single layer of polyethylene. Alternatively, the second component 320 may also include a laminate of two or more layers.

As shown in Figure 3, the second component 320 is bonded to the inner surface of the topsheet 24. The second component 320 may also be bonded to the outer surface of the topsheet 24. Alternatively, the second component 320 may also be bonded to either the outer or inner surface of the backsheet 26.

In another embodiment, the composite forming the elastic waist feature 34 may comprise a portion of the topsheet 24 and a portion of the backsheet 26. For example, a portion of the topsheet 24, while in a relaxed condition, is intermittently bonded to a portion of the backsheet 26 which is the second SELF web and is in a stretched or elongated condition. Alternatively, the topsheet 24 may form the second SELF web, while the backsheet 26 forms the first web of the composite.

It has been found that the extension characteristics, including the extension forces, an extension modulus, and available stretch (extension), and the contractive forces, elastic creep, elastic hysteresis, and rate of contraction of the composite are important considerations in the performance of the elastic waist feature 34 and other portions of the diaper 20. The extension and contraction characteristics of the composite give the diaperer and wearer an overall perceived "stretchiness" during use. They also effect the ability of the diaper to achieve a suitable degree of application stretch (i.e., for a "normally" perceived tension of the diaper during application, the total amount of resultant stretch is that desired to achieve/maintain good conformity of fit). For example, a waist feature with a relatively high available stretch and low extension modulus can cause gapping and sagging/slipping on the wearer. A waist feature having too little available stretch or too high of an extension modulus may not achieve a suitable level of body conformity and may contribute in making the diaper uncomfortable to wear and hard to apply to the wearer.

Typical extensible materials show a hysteresis loop of force in their stress-strain property. That is, for a given extension, the force (extension force) required to uniaxially extend the extensible material is greater than the force (contractive force) the extensible material exerts when it is allowed to contract from its pre-extended condition. The former curve can be referred to as the "load curve" and the latter curve can be referred to as the "unload curve". The "load" extension force (extension force) is felt by the diaperer when the extensible waist feature is stretched to apply the diaper to the wearer. The wearer more nearly "feels" the "unload" forces (contractive forces) once the diaper is on. Therefore, the hysteresis loss should not be so great that the contractive force is low enough to allow sagging/gapping of the diaper on the wearer.

The extensible composite may also be provided with differential extensibility. As used herein, the term "differential extensibility" is used to mean a material having a non-uniform degree of extensional properties, as measured in the direction of stretching at various points along an axis oriented substantially perpendicular to the direction of stretching. This may, for example, include varying the elastic modulus or available stretch or both of the extensible composite. The differential extensibility can be achieved in a number of different ways. The extensible composite can have multiple combined extensible materials, multiple configurations for the extensible materials, or the extension properties of the extensible or other materials making up the extensible composite may be non-uniform. For example, differential extensibility can be achieved in selected adjacent portions of the extensible composite by using extensible materials having varying extensions or contractive forces, modulus, or other inherent properties such that more or less "varying" lateral extensibility is achieved in one portion of the extensible composite versus another. The extensible materials may also have varying length, sizes, and shapes that provide differential extensibility. Other ways of varying the properties of materials that form the extensible composite that are known in the art may also be used.

Similar to the elastic waist feature 34, the extensible side panels 30 and the elasticized leg cuffs 32 may also be comprised of a first web intermittently bonded to a SELF web while the first web is in a relaxed condition and the SELF web is in a stretched or elongated condition. In addition, other portions of the diaper 20 such as the absorbent core, the fastening member, the backsheet, the topsheet, portions of the backsheet or topsheet, or the entire diaper may also be made extensible by constructing them from the composite of the present invention.

Additionally, the first web of the composite may also be a SELF web similar to the second SELF web. However, only one of the SELF webs will be in a stretched or elongated condition when intermittently bonded to the other SELF web which is in a relaxed condition.

The diaper 20 is also preferably provided with a fastening system 48 for fitting the diaper on the wearer. The fastening system 48 maintains the first waist region 50 and the second waist region 52 in an overlapping configuration to form a side closure. The fastening system 48 further maintains tension in the extensible side panels 30 to hold the diaper 20 on the wearer as well as to provide for improved dynamic fit about the legs and waist of the wearer. The fastening system 48 may comprise any attachment means known in the art, including, but not limited to, pressure sensitive adhesives, cohesive materials, mechanical fastening means, such as hook and loop type fasteners, or any combination of these or any other attachment means as known in the art. Examples of suitable adhesive tape tab fastening systems are disclosed in U.S. Patent 3,848,594 issued to Buell on November 19, 1974; and U.S. Patent 4,662,875 issued to Hirotsu and Robertson on May 5, 1987. Examples of other closure systems, including mechanical closure systems, useful in the present invention are disclosed in U.S. Patent 4,869,724 issued to Scripps on September 26, 1989; U.S. Patent 4,848,815 issued to Scripps on July 11, 1989; and U.S. Patent 5,242,436 issued to Weil, Buell, Clear, and Falcone on September 7, 1993.

The diaper 20 is preferably applied to a wearer by positioning one of the waist regions, preferably the first waist region 50, under the wearer's back and drawing the remainder of the diaper 20 between the wearer's legs so that the other waist region, preferably the second waist region 52, is positioned across the front of the wearer. The diaperer then wraps one extensible side panel 30 around the wearer, while grasping one of the first fastening members 40 disposed on each of the extensible side panels 30. The diaperer then repeats this step for the other extensible side panel 30. The waist closure is formed by engagement of the first fastening members 40 to the second fastening member 42 located in the second waist region 52. With the formation of the waist closure, the diaper 20 is initially conformably fit about the wearer. If the diaper 20 has been fitted asymmetrically, the diaper 20 will self-adjust during wear to attain an improved fit. Once fitted to the wearer, the extensible side panels 30 and the elastic waist feature 34 expand and contract in conjunction with the motions of the wearer to provide improved sustained dynamic fit throughout the time of wear, well past when the diaper 20 has been loaded with exudates. This improved dynamic fit reduces sagging and gapping of the diaper 20 in the waist and thigh regions while increasing wearer comfort.

## Claims

1. A composite (200) exhibiting an elastic-like behavior in response to an applied axial elongation along at least one axis thereof said composite comprising: a first web (250) material in a relaxed condition bonded to a stretched second web material (252), said second web material (252) characterized by a strainable network having first and second regions (264 and 266) formed of substantially the same material composition, said first region (264) providing a first, elastic-like resistive force to said applied axial elongation, and said second region (266) providing a second distinctive resistive force to further applied axial elongation, thereby providing at least two stages of resistive forces in use.

2. The composite (200) of claim 1 wherein said first region (264) undergoes a substantially molecular-level deformation and said second region (266) initially undergoes a substantially geometric deformation when said web material (252) is subjected to said applied axial elongation.

3. The composite (200) of either Claim 1 or Claim 2 wherein said first web material (250) and said second web material (252) comprise different materials.

4. The composite (200) of any one of the preceding claims wherein said first web material (250) is pre-strained.

5. The composite (200) of any one of the preceding claims wherein said first web material (250) is intermittently bonded to said second web material (252).

6. The composite (200) of any one of the preceding claims wherein said first web material (250) comprises a laminate of two or more materials.

7. The composite (200) of any one of the preceding claims wherein said second web material (252) comprises a laminate of two or more materials.

8. The composite (200) of any one of the preceding claims wherein said composite (200) forms a portion of a disposable absorbent article (20).

9. The composite (200) of Claim 8 wherein said composite (200) forms an elastic waist feature (34) on a disposable diaper (20)

10. The composite (200) of Claim 8 wherein said composite (200) forms an extensible side panel (30) on a disposable diaper (20).

11. The composite (200) of Claim 8 wherein said composite (200) forms an elasticized leg cuff (32) on a disposable diaper (20).

12. The composite (200) of Claim 8 wherein said composite (200) forms at least a portion of a backsheet (26) on a disposable diaper (20).

13. The composite (200) of Claim 8 wherein said composite (200) forms at least a portion of a fastening member (48).

## Patentansprüche

1. Schichtkörper (200) mit einem elastikähnlichen Verhalten als Reaktion auf eine beaufschlagte axialen Längung entlang wenigstens einer Achse desselben, wobei der Schichtkörper umfaßt: ein erstes Bahnmaterial (250) in einem entspannten Zustand, das mit einem gedehnten zweiten Bahnmaterial (252) verbunden ist, wobei das zweite Bahnmaterial (252) gekennzeichnet ist durch ein streckbares Netzwerk mit einem ersten und einem zweiten Bereich (264 und 266), die aus im wesentlichen der gleichen Materialzusammensetzung gebildet sind, wobei der erste Bereich (264) eine erste, elastikähnliche Widerstandskraft gegenüber der beaufschlagten axialen Längung liefert und der zweite Bereich (266) eine zweite, unterschiedliche Widerstandskraft gegenüber einer weitergehenden beaufschlagten Längung liefert, wodurch bei der Benutzung für wenigstens zwei Stufen von Widerstandskräften gesorgt wird.

2. Schichtkörper (200) nach Anspruch 1, in welchem der erste Bereich (264) eine Verformung im wesentlichen auf Molekularebene erfährt und der zweite Bereich (266) anfänglich eine im wesentlichen geometrische Verformung erfährt, wenn das Bahnmaterial (252) der beaufschlagten axialen Längung unterzogen wird.

3. Schichtkörper (200) nach Anspruch 1 oder Anspruch 2, in welchem das erste Bahnmaterial (250) und das zweite Bahnmaterial (252) unterschiedliche Materialien umfaßt.

4. Schichtkörper (200) nach einem der vorstehenden Ansprüche, in welchem das erste Bahnmaterial (250) vorgestreckt ist.

5. Schichtkörper (200) nach einem der vorstehenden Ansprüche, in welchem das erste Bahnmaterial (250) mit dem zweiten Bahnmaterial (252) intermittierend verbunden ist.

6. Schichtkörper (200) nach einem der vorstehenden Ansprüche, in welchem das erste Bahnmaterial (250) ein Laminat aus zwei oder mehr Materialien umfaßt.

7. Schichtkörper (200) nach einem der vorstehenden Ansprüche, in welchem das zweite Bahnmaterial (252) ein Laminat aus zwei oder mehr Materialien umfaßt.

8. Schichtkörper (200) nach einem der vorstehenden Ansprüche, in welchem der Schichtkörper (200) einen Teil eines absorbierenden Einwegartikels (20) bildet.

9. Schichtkörper (200) nach Anspruch 8, in welchem der Schichtkörper (200) ein elastisches Hüftmerkmal (34) an einer Einwegwindel (20) bildet.

10. Schichtkörper (200) nach Anspruch 8, in welchem der Schichtkörper (200) ein dehnbares Seitenfeld (30) an einer Einwegwindel (20) bildet.

11. Schichtkörper (200) nach Anspruch 8, in welchem der Schichtkörper (200) einen elastisch gemachten Beinaufschlag (32) an einer Einwegwindel (20) bildet.

12. Schichtkörper (200) nach Anspruch 8, in welchem der Schichtkörper (200) wenigstens einen Teil einer Hinterschicht (26) an einer Einwegwindel (20) bildet.

13. Schichtkörper (200) nach Anspruch 8, in welchem der Schichtkörper (200) wenigstens einen Teil eines Befestigungselements (48) bildet.

## Revendications

1. Composite (200) présentant un comportement de type élastique en réponse à un allongement axial appliqué, le long d'au moins l'un de ses axes, ledit composite comprenant : un premier matériau de nappe (250) dans un état relâché, lié à un deuxième matériau de nappe (252) étiré, ledit deuxième matériau de nappe (252) étant caractérisé par un réseau défonnable ayant des première et deuxième régions (264 et 266) formées essentiellement de la même composition de matériau, ladite première région (264) fournissant une première force de résistance, de type élastique, audit allongement axial appliqué, et ladite deuxième région (266) fournissant une deuxième force de résistance distincte à un autre allongement axial appliqué, ce qui crée au moins deux étapes de forces de résistance en utilisation.

2. Composite (200) selon la revendication 1, dans lequel ladite première région (264) subit une déformation essentiellement au niveau moléculaire et ladite deuxième région (266) subit initialement une déformation essentiellement géométrique lorsque ledit matériau de nappe (252) est soumis audit allongement axial appliqué.

3. Composite (200) selon la revendication 1 ou la revendication 2, dans lequel ledit premier matériau de nappe (250) et ledit deuxième matériau de nappe (252) sont constitués de matériaux différents.

4. Composite (200) selon l'une quelconque des revendications précédentes, dans lequel ledit premier matériau de nappe (250) est pré-déformé.

5. Composite (200) selon l'une quelconque des revendications précédentes, dans lequel ledit premier matériau de nappe (250) est lié d'une manière intermittente audit deuxième matériau de nappe (252).

6. Composite (200) selon l'une quelconque des revendications précédentes, dans lequel ledit premier matériau de nappe (250) comprend un stratifié de deux ou plusieurs matériaux.

7. Composite (200) selon l'une quelconque des revendications précédentes, dans lequel ledit deuxième matériau de nappe (252) comprend un stratifié de deux ou plusieurs matériaux.

8. Composite (200) selon l'une quelconque des revendications précédentes, dans lequel ledit composite (200) forme une partie d'un article absorbant jetable (20).

9. Composite (200) selon la revendication 8, dans lequel ledit composite (200) forme un élément de ceinture élastique (34) sur une couche jetable (20).

10. Composite (200) selon la revendication 8, dans lequel ledit composite (200) forme un panneau latéral extensible (30) sur une couche jetable (20).

11. Composite (200) selon la revendication 8, dans lequel ledit composite (200) forme un revers de jambe élastifié (32) sur une couche jetable (20).

12. Composite (200) selon la revendication 8, dans lequel ledit composite (200) forme au moins une partie d'une feuille de fond (26) sur une couche jetable (20).

13. Composite (200) selon la revendication 8, dans lequel ledit composite (200) forme au moins une partie d'un organe de fixation (48).
